# EUROPÄISCHE PATENTANMELDUNG

(11) **EP 4 374 846 A1**
(43) Veröffentlichungstag der Anmeldung: **29.05.2024**
(21) Anmeldenummer: 23204063.4
(22) Anmeldetag: 17.10.2023
(51) Int. Cl.: A61K 8/73, A61K 8/92, A61Q 1/02

(54) **TÄTOWIERFARBE**

(30) Priorität: 22.11.2022 DE 102022130838
(71) Anmelder: Veil VariEty In coLours GmbH, 22453 Hamburg (DE)
(72) Erfinder: Wolde, Maximilian Tim, 22301 Hamburg (DE)
(74) Vertreter: Jostarndt Patentanwalts-AG

(57) **Zusammenfassung**

Die Erfindung betrifft eine Tätowierfarbe mit verbesserter Hautverträglichkeit basierend auf einer Dispersion mindestens eines Pigments in einer wässrigen Trägerflüssigkeit, die ein Ingwer-Öl und/oder Cajeputöl und Hyaluron und/oder ein oder mehrere Hyaluronderivaten enthält, deren Verwendung und ein Verfahren zur Herstellung von Tätowierungen.

## Beschreibung

Die Erfindung betrifft eine Tätowierfarbe (häufig auch als Tätowiertinte oder Tätowiermittel bezeichnet) mit verbesserter Hautverträglichkeit.

Eine Tätowierung ist ein Motiv, das mittels einer Tätowierfarbe oder anderen Farbmitteln in die Haut eines Lebewesens eingebracht wird. Das Motiv ist beliebig und kann ein einfarbiges oder mehrfarbiges Bild, Zeichen oder Symbol, Muster, Ornament oder auch Text darstellen. Die Tätowierung kann bei Menschen und Tieren durchgeführt werden.

Tätowierungen beim Menschen können als Körperverzierungen, Permanent-Make-up, als Schmuck, Zeichen der Zugehörigkeit zu einer Gruppe, als Statement oder Code oder als modisches Accessoire dienen. Auch im medizinischen Bereich können Tätowierungen eingesetzt werden, beispielweise zum Überdecken von Narbengewebe oder bei Brustrekonstruktionen. Bei Haus- und Zuchttieren dient eine Tätowierung in der Regel zur Kennzeichnung und/oder Identifizierung.

Bei der Tätowierung wird eine Tätowierfarbe, je nach dem gewünschten Effekt, durch eine oder mehrere Nadeln in die zweite Hautschicht (die Lederhaut) gestochen. Während sich die darüber liegende oberste Hautschicht ständig erneuert, tut dies die Lederhaut nicht. Die Farbpigmente aus der Tätowierfarbe werden dort dauerhaft eingelagert, so dass das tätowierte Motiv dauerhaft sichtbar bleibt. Das Tätowieren stellt eine Punktierung der Haut dar, wobei gleichzeitig mit dem Durchstechen ein mittels der Tätowierfarbe Färbemittel in die Haut eingebracht wird.

Zum Tätowieren benutzen Tattoo-Studios in der Regel eine elektrische Tätowiermaschine. Damit wird mit speziell geschliffenen Nadelspitzen die oberste Schicht oder Epidermis der Haut durchstochen und die Tätowierfarbe in die zweite Schicht oder Lederhaut eingebracht. Der Einstich darf nicht nur oberflächlich und auch nicht zu tief sein. Wird die Tätowierfarbe nur in die Epidermis eingebracht, so würde aufgrund ständiger Erneuerung dieser Hautschicht das Färbemittel schrittweise abgetragen. Wird zu tief gestochen, kommt es durch die auftretenden Blutungen zu einem Auswaschen der Färbemittel. Daher ist beim Tätowieren Einstellung der Einstichtiefe in Abhängigkeit vom Hauttyp und der zu tätowierenden Körperstelle erforderlich.

Übliche Tätowierfarben umfassen ein oder mehrere Färbemittel, ein oder mehrere Trägerflüssigkeiten und gegebenenfalls Hilfsstoffe. Trägerflüssigkeiten sind Lösungs- und/oder Dispergiermittel wie beispielsweise destilliertes Wasser oder Alkohole.

Als Färbemittel sind in Tätowierfarben Farbpigmente, beispielsweise Metallsalze, enthalten. Diese können wasserlöslich sein, sind aber häufig hydrophob und damit nicht wasserlöslich, so dass Lösungsvermittler, Lösungshilfsmittel und/oder Dispergiermittel eingesetzt werden, um die Pigmente in Wasser zu dispergieren.

Da die Tätowierfarbe zu einem wesentlichen Teil im Körper verbleibt, ist deren Qualität von besonderer Bedeutung.

Ein Problem beim Tätowieren ist, dass das Eindringen der Nadel in die Haut als unangenehm oder sogar schmerzhaft empfunden wird und darüber hinaus häufig auch zu Hautreaktionen führt. Es hat daher nicht an Versuchen gefehlt, Tätowierfarben zu entwickeln, die den Schmerz und die Hautreaktionen beim und nach dem Tätowiervorgang reduzieren.

So beschreibt die WO 2019/057822 eine Kombination zum Tätowieren, umfassend eine Tätowiertinte und ein oder mehrere Lokalanästhetika für die Haut.

KR102084261B1 offenbart eine Tätowierfarbe und ein Verfahren zu deren Herstellung, wobei die Tätowierfarbe verschiedene Pflanzenextrakte enthält, die der Tätowierfarbe antibakterielle, antientzündliche und schmerzlindernde Eigenschaften verleihen sollen.

Dennoch besteht nach wie vor Bedarf für Tätowierfarben, die den Schmerz und die Hautreaktionen beim und nach dem Tätowiervorgang weiter reduzieren.

Aufgabe der Erfindung ist es daher, Tätowierfarben bereitzustellen, die gesundheitlich unbedenklich sind und den Schmerz und die Hautreaktionen beim und nach dem Tätowiervorgang weiter reduzieren.

Erfindungsgemäß wird diese Aufgabe gelöst durch eine Tätowierfarbe mit den Merkmalen des unabhängigen Anspruches 1, deren Verwendung gemäß Anspruch 15 und 16 sowie ein Verfahren zur Herstellung von Tätowierungen gemäß Anspruch 17. Vorteilhafte Weiterbildungen ergeben sich aus den Unteransprüchen 2 bis 14.

Die erfindungsgemäße Tätowierfarbe basiert auf einer Dispersion mindestens eines Pigments in einer wässrigen Trägerflüssigkeit und enthält eine Kombination aus mindestens einem Öl, das ausgewählt ist aus einem Ingwer-Öl und Cajeputöl, und aus Hyaluron und/oder einem oder mehreren Hyaluronderivaten. Dabei beträgt die Summe der Anteile des mindestens einen Öls und des Hyalurons und/oder eines oder mehrerer Hyaluronderivate an der erfindungsgemäßen Tätowierfarbe vorzugsweise 0,1 bis 11 Gew.-%, insbesondere 1 bis 8 Gew.-% und besonders bevorzugt bei 2,5 bis 7 Gew.-%. Im Einzelnen enthält die Tätowierfarbe 0,05 bis 10 Gew.-%, vorzugsweise 0,05 bis 7 Gew.-% und insbesondere 0,5 bis 5 Gew.-% Ingwer-Öl und/oder 0,01 bis 10 Gew.-%, vorzugsweise 0,01 bis 7 Gew.-% und insbesondere 0,5 bis 3 Gew.-% Cajeputöl und 0,01 bis 10 Gew.-%, vorzugsweise 0,01 bis 7 Gew.-% und insbesondere 0,5 bis 5 Gew.-% Hyaluron und/oder ein oder mehrere Hyaluronderivate.

In einer besonders bevorzugten Ausführungsform enthält die erfindungsgemäße Tätowierfarbe ein Ingwer-Öl, Cajeputöl und Hyaluron und/oder ein oder mehrere Hyaluronderivate.

Unter Ingwer-Öl werden dabei ätherische Öle verstanden, die aus einer Ingwerwurzel gewonnen wurden. Derartige Öle werden in kosmetischer Qualität von zahlreichen Anbietern kommerziell angeboten. Erfindungsgemäß besonders bevorzugt ist das Öl von Ingwer Cassumunar, auch bekannt als Plaiöl.

Bei Cajeputöl handelt es sich um ein ätherisches Öl, das aus den Blättern und Zweigspitzen des Cajeputbaumes (Malealeuca leucadendra) gewonnen wird. Auch dieses ätherische Öl ist in kosmetischer Qualität kommerziell erhältlich.

Hyaluron bezeichnet die Hyaluronsäure und ihre physiologisch verträglichen Salze. Dabei wird erfindungsgemäß vorzugsweise Natrium-Hyaluronat eingesetzt. Dabei sind alle Molekulargewichte, die biotechnologisch herstellbar sind, einsetzbar, bevorzugt Hyaluronsäure mit einem Molekulargewicht <5 Kilodalton, 10-50 Kilodalton, 100-300 Kilodalton, 400-800 Kilodalton, 1200-1800 Kilodalton, sowie über 2200 Kilodalton.

Hyaluronderivate bezeichnet Verbindungen der Hyaluronsäure und insbesondere Hydroxypropyl Trimonium Hyaluronate, Hydrolysiertes Natrium-Hyaluronate, vegane Hyaluronsäure Tremella Fuciformis Sporocarp Extract, Natriumacetyliertes Hyaluronat und Natrium Hyaluronate Crosspolymer Bei Einsatz von Hyaluronderivaten kann es erfindungsgemäß vorteilhaft sein, wenn die Tätowierfarbe zusätzlich N-Acetylglucosamin, einen Baustein der Hyaluronsäure, enthält. In dieser Ausführungsform beträgt der Anteil an N-Acetylglucosamin an der Tätowierfarbe vorzugsweise 0,5 bis 5 Gew.-%.

Außerdem können in der erfindungsgemäßen Tätowierfarbe übliche Hilfsstoffe mit einem Anteil von bis zu 30 Gew.-% enthalten sein. Dabei handelt es sich vorzugsweise um Befeuchtungsmittel, insbesondere Pentylenglykol oder Panthenol, Dispergiermittel, insbesondere Polyhydroxystearinsäure, Bindemittel, Verdickungsmittel, Viskositatsregulatoren, Konservierungsmittel, Stabilisatoren, Emulgatoren, Antioxidationsmittel, oder Mittel zur Regulierung des pH-Werts.

Bei der wässrigen Trägerflüssigkeit handelt es sich um Wasser, ein pflanzliches Hydrolat oder um ein Gemisch aus Wasser oder einem pflanzlichen Hydrolat und einem oder mehreren physiologisch unbedenklichen Lösungsmitteln, insbesondere niederen gesättigten Alkoholen oder besonders bevorzugt Glycerin, wobei der Anteil des Wassers oder des pflanzlichen Hydrolats an dem Gemisch vorzugsweise mindestens 70 Gew.-% und insbesondere mindestens 80 Gew.-% beträgt. Der Anteil der wässrigen Trägerflüssigkeit an der Tätowierfarbe beträgt vorzugsweise 10 bis 98 Gew.-%, besonders bevorzugt 30 bis 90 Gew.-% und insbesondere 40 bis 70 Gew.-%. Dabei ist es zweckmäßig, die wässrige Trägerflüssigkeit und ihre Bestandteile in steriler Form einzusetzen.

Der Anteil des mindestens einen Pigments an der Tätowierfarbe beträgt vorzugsweise 1 bis 90 Gew.-%, besonders bevorzugt 5 bis 40 Gew.-%und insbesondere 10 bis 40 Gew.-%.

Erfindungsgemäß geeignete Pigmente sind anorganische Farbpigmente, sowie Weißpigmente aus der Gruppe Titandioxid, Zinkoxide und Bornitrid. Bevorzugte Pigmente sind Eisenoxide, Aluminiumoxid, Zirkoniumoxide, Kobaltoxide, Ceroxide, Nickeloxid, Nickel-Chrom-Oxide, sowie zusammengesetzte Oxide oder Calciumhydroxid, Eisenhydroxide, Aluminiumhydroxid, Chromhydroxid, Magnesiumhydroxid sowie zusammengesetzten Metallhydroxide, außerdem Ultramarinblau, Eisensulfide, Manganviolett, Ruß, Glimmer oder Kaolin.

In einer Ausführungsform der Erfindung wird als Pigment ein Weißpigment aus der Gruppe Titandioxid, Zinkoxide und Bornitrid als Trägerpigment in Kombination mit mindestens einem anorganischen Farbpigment eingesetzt. Dabei ist Bornitrid als Weißpigment besonders bevorzugt.

Trägerpigment bedeutet dabei, dass das Trägerpigment mit dem anorganischen Farbpigment beschichtet ist.

Eine erfindungsgemäße Tätowierfarbe kann vorzugsweise folgende Zusammensetzung aufweisen:
40 bis 70 Gew.-% wässrige Trägerflüssigkeit 2,5 bis 8 Gew.-% der Kombination aus Ingwer-Öl, Cajeputöl und Hyaluron10 bis 40 Gew.-% mindestens eines Pigments 0 bis 15 Gew.-% Hilfsstoffe
Die Zusammensetzung der erfindungsgemäßen Tätowierfarbe kann in einem breiten Bereich an unterschiedliche Anforderungen beim Tätowieren angepasst werden. Insbesondere lässt sich die Viskosität in einem breiten Bereich einstellen.

Dabei bewirkt die Kombination aus Ingwer-Öl und/oder Cajeputöl und Hyaluron und/oder einen oder mehreren Hyaluronderivaten nach dem Tätowiervorgang eine verbesserte Hautregeneration und wirkt abschwellend und entzündungshemmend. Besonders vorteilhafte Wirkungen werden dabei erzielt, wenn die Tätowierfarbe ein Ingwer-Öl, insbesondere Plaiöl, Cajeputöl und Hyaluron und/oder ein oder mehrere Hyaluronderivate enthält.

Die erfindungsgemäße Tätowierfarbe ist besonders geeignet zur Herstellung einer permanenten Tätowierung auf der Haut eines Lebewesens, insbesondere eines Menschen.

Darüber hinaus eignet sich die erfindungsgemäße Tätowierfarbe auch Verwendung als "Leave on" Farbe zur vorübergehenden Färbung der Haut eines Lebewesens, insbesondere eines Menschen, oder von Teilen davon.

Die erfindungsgemäße Tätowierfarbe lässt sich in dem Fachmann bekannter Weise durch Mischen der Bestandteile herstellen.

Gegenstand der Erfindung ist auch ein Verfahren zur Herstellung von Tätowierungen, bei dem eine geeignete Menge der erfindungsgemäßen Tätowierfarbe auf die Nadeln einer Tätowiermaschine, insbesondere einer Magnetspulenmaschine oder einer Rotationstätowiermaschine, aufgebracht und mittels der Tätowiermaschine in die Haut eines Lebewesens, insbesondere eines Menschen, eingebracht wird.

Weitere Vorteile, Besonderheiten und zweckmäßige Weiterbildungen der Erfindung ergeben sich aus den nachfolgenden Ausführungsbeispielen.

### Beispiele:

| | Beispiel 1 | Beispiel 2 | Beispiel 3 | Beispiel 4 | Beispiel 5 |
|---|---|---|---|---|---|
| Rohstoff | Gew% | Gew% | Gew% | Gew% | Gew% |
| Glycerin 99,5% Vegan | 6 | 5 | 5 | 5 | 5 |
| Wasser, demin. | 41 | 45,5 | 45,5 | 45,5 | 32,5 |
| Plaiöl | 1 | 1 | 1 | 1 | 4 |
| Cajeputöl | 1 | 1 | 1 | 2 | 1 |
| Pentylenglycol | 5 | 8 | 8 | 5 | 8 |
| Panthenol | 5 | 3 | 3 | 3 | 3 |
| Natrium Hyaluronat < 50 Kilodalton | 5 | 0,5 | 0,5 | 2,5 | 0,5 |
| Polyhydroxystearinsäure | 1 | 1 | 1 | 1 | 1 |
| Eisenoxid schwarz CI 77499 | 35 | | | | |
| Cromoxid grün CI 77288 | | 35 | | | |
| Zinkoxid Cl 77947, Boron Nitride | | | 35 | | |
| Eisenoxid gelb Cl 77492 | | | | 35 | |
| Ultramarinblau Cl 77007, Kaolin | | | | | 45 |

| | Beispiel 6 | Beispiel 7 | Beispiel 8 | Beispiel 9 | Beispiel 10 |
|---|---|---|---|---|---|
| Rohstoff | Gew% | Gew% | Gew% | Gew% | Gew% |
| Glycerin 99,5% Vegan | 6 | 5 | 5 | 5 | 5 |
| Wasser, demin. | 45 | 45,5 | 45 | 45,5 | 22,75 |
| Plaiöl | 1 | 1 | 1 | 1 | 4 |
| Cajeputöl | 1 | 1 | 1 | 2 | 1 |
| Pentylenglycol | 5 | 8 | 8 | 5 | 8 |
| Panthenol | 5 | 3 | 3 | 3 | 3 |
| Natrium Hyaluronat < 50 Kilodalton | | 0.5 | | | |
| Natrium Hyaluronat 1800 Kilodalton | 1 | | | | |
| Natrium Hyaluronat 400 Kilodalton | | | 1 | | |
| Hydrolisiertes Natrium Hyaluronat | | | | 2,5 | |
| Natrium Hyaluronat Crosspolymer | | | | | 0,25 |
| Polyhydroxystearinsäure | 1 | 1 | 1 | 1 | 1 |
| Eisenoxid schwarz CI 77499 | 35 | | | | |
| Eisenoxid rot CI 77491, Eisenoxid schwarz CI 77499 | | 35 | | | |
| Zinkoxid CI 77947, Bornitrid | | | 35 | | |
| Eisenoxid gelb CI 77492 | | | | 35 | |
| Ultramarinblau CI 77007, Kaolin | | | | | 55 |

| | Beispiel 11 | Beispiel 12 | Beispiel 13 | Beispiel 14 | Beispiel 15 |
|---|---|---|---|---|---|
| Rohstoff | Gew% | Gew% | Gew% | Gew% | Gew% |
| Glycerin 99,5% Vegan | 6 | 5 | 5 | 5 | 5 |
| Wasser, demin. | 45 | 53,5 | 33 | 40,5 | 42,75 |
| Plaiöl | 0,5 | 1 | 5 | 1 | |
| Cajeputöl | 0,5 | 3 | 1 | 2 | 1 |
| Pentylenglycol | 5 | 8 | 8 | 5 | 8 |
| Panthenol | 5 | 3 | 1 | 3 | 4 |
| Natrium Hyaluronat < 50 Kilodalton | | 0,5 | | | |
| Natrium Hyaluronat 1800 Kilodalton | | | | | |
| Natrium Hyaluronat 400 Kilodalton | | | 1 | | |
| Hydrolisiertes Natrium Hyaluronat | | | | 2,5 | |
| Natrium Hyaluronat Crosspolymer | 2 | | | | 0,25 |
| Acetylglucosamin | | | | | 3 |
| Polyhydroxystearinsäure | 1 | 1 | 1 | 1 | 1 |
| Eisenoxid schwarz CI 77499 | 35 | | | | |
| Eisenoxid rot CI 77491, Eisenoxid schwarz CI 77499 | | 25 | | | |
| Zinkoxid CI 77947, Bornitrid | | | 45 | | |
| Eisenoxid gelb CI 77492 | | | | 40 | |
| Ultramarinblau CI 77007, Kaolin | | | | | 35 |

Die Farbe wird im One-Pot-Verfahren hergestellt, d.h. alle Rohstoffe werden zusammen eingewogen und bei 25°C mit einem UltraTurrax T25 der Firma IKA für eine Minute bei 5000 U/Min homogenisiert. Danach ist die Tätowierfarbe fertig und kann verwendet werden.

Die hier gezeigten Ausführungsformen stellen nur Beispiele für die vorliegende Erfindung dar und dürfen daher nicht einschränkend verstanden werden. Alternative durch den Fachmann in Erwägung gezogene Ausführungsformen sind gleichermaßen vom Schutzbereich der vorliegenden Erfindung umfasst.

## Patentansprüche

1. Tätowierfarbe basierend auf einer Dispersion mindestens eines Pigments in einer wässrigen Trägerflüssigkeit,
**dadurch gekennzeichnet,**
**dass** die Tätowierfarbe eine Kombination aus mindestens einem Öl, das ausgewählt ist aus einem Ingwer-Öl und Cajeputöl, und aus Hyaluron und/oder einem oder mehreren Hyaluronderivate enthält.

2. Tätowierfarbe gemäß Anspruch 1,
**dadurch gekennzeichnet,**
**dass** die Summe der Anteile des mindestens einen Öls und von Hyaluron und/oder einem oder mehreren Hyaluronderivaten an der Tätowierfarbe 0,1 bis 11 Gew.-%, insbesondere bei 1 bis 8 Gew.-% und besonders bevorzugt bei 2,5 bis 7 Gew.-% beträgt.

3. Tätowierfarbe gemäß Anspruch 1 oder 2,
**dadurch gekennzeichnet,**
**dass** die Tätowierfarbe 0,05 bis 10 Gew.-%, vorzugsweise 0,05 bis 7 Gew.-% und insbesondere 0,5 bis 5 Gew.-% Ingwer-Öl und/oder 0,01 bis 10 Gew.-%, vorzugsweise 0,01 bis 7 Gew.-% und insbesondere 0,5 bis 3 Gew.-% Cajeputöl und 0,01 bis 10 Gew.-%, vorzugsweise 0,01 bis 7 Gew.-% und insbesondere 0,5 bis 5 Gew.-% Hyaluron und/oder ein oder mehrere Hyaluronderivate enthält.

4. Tätowierfarbe gemäß einem der Ansprüche 1 bis 3,
**dadurch gekennzeichnet,**
**dass** die Tätowierfarbe ein Ingwer-Öl, Cajeputöl und Hyaluron und/oder ein oder mehrere Hyaluronderivate enthält.

5. Tätowierfarbe gemäß einem der Ansprüche 1 bis 4,
**dadurch gekennzeichnet,**
**dass** das Hyaluron Hyaluronsäure oder ein physiologisch verträgliches Salz der Hyaluronsäure, vorzugsweise Natrium-Hyaluronat ist.

6. Tätowierfarbe gemäß einem der Ansprüche 1 bis 4,
**dadurch gekennzeichnet,**
**dass** die ein oder mehreren Hyaluronderivate ausgewählt sind aus der Gruppe Hydroxypropyl Trimonium Hyaluronate, Hydrolysiertes Natrium-Hyaluronate, vegane Hyaluronsäure Tremella Fuciformis Sporocarp Extract, Natriumacetyliertes Hyaluronat und Natrium Hyaluronate Crosspolymer.

7. Tätowierfarbe gemäß einem der Ansprüche 1 bis 4 oder 6,
**dadurch gekennzeichnet,**
**dass** die Tätowierfarbe ein oder mehrere Hyaluronderivate und N-Acetylglucosamin enthält.

8. Tätowierfarbe gemäß Anspruch 7,
**dadurch gekennzeichnet,**
**dass** die die Tätowierfarbe 0,5 bis 5 Gew.-% N-Acetylglucosamin enthält.

9. Tätowierfarbe gemäß einem der vorherigen Ansprüche,
**dadurch gekennzeichnet,**
**dass** das Ingwer-Öl Plaiöl ist.

10. Tätowierfarbe gemäß einem der vorherigen Ansprüche,
**dadurch gekennzeichnet,**
**dass** es sich bei der wässrigen Trägerflüssigkeit um Wasser, ein pflanzliches Hydrolat oder um ein Gemisch aus Wasser oder einem pflanzlichen Hydrolat und einem oder mehreren physiologisch unbedenklichen Lösungsmitteln, insbesondere niederen gesättigten Alkoholen oder besonders bevorzugt Glycerin handelt.

11. Tätowierfarbe gemäß einem der vorherigen Ansprüche,
**dadurch gekennzeichnet,**
**dass** der Anteil des mindestens einen Pigments an der Tätowierfarbe 1 bis 90 Gew.-%, besonders bevorzugt 5 bis 50 Gew.-% und insbesondere 10 bis 45 Gew.-% beträgt.

12. Tätowierfarbe gemäß einem der vorherigen Ansprüche
**dadurch gekennzeichnet,**
**dass** die Tätowierfarbe als Pigment ein Weißpigment aus der Gruppe Titandioxid, Zinkoxide und Bornitrid als Trägerpigment in Kombination mit mindestens einem anorganischen Farbpigment enthält.

13. Tätowierfarbe gemäß Anspruch 12
**dadurch gekennzeichnet,**
**dass** die Tätowierfarbe als Trägerpigment Bornitrid enthält

14. Tätowierfarbe gemäß einem der vorherigen Ansprüche,
**dadurch gekennzeichnet,**
**dass** sie eine Zusammensetzung aus
40 bis 70 Gew.-% wässrige Trägerflüssigkeit
2,5 bis 8 Gew.-% der Kombination aus Ingwer-Öl, Cajeputöl und Hyaluron und/oder einem oder mehreren Hyaluronderivaten
10 bis 40 Gew.-% mindestens eines Pigments
0 bis 15 Gew.-% Hilfsstoffe
aufweist.

15. Verwendung einer Tätowierfarbe gemäß einem der Ansprüche 1 bis 14 zur Herstellung einer permanenten Tätowierung auf der Haut eines Lebewesens.

16. Verwendung einer Tätowierfarbe gemäß einem der Ansprüche 1 bis 14 als Leave on Farbe zur vorübergehenden Färbung der Haut eines Lebewesens oder von Teilen davon.

17. Verfahren zur Herstellung von Tätowierungen,
**dadurch gekennzeichnet,**
**dass** eine geeignete Menge einer Tätowierfarbe gemäß einem der Ansprüche 1 bis 14 auf die Nadeln einer Tätowiermaschine, insbesondere einer Magnetspulenmaschine oder einer Rotationstätowiermaschine, aufgebracht und mittels der Tätowiermaschine in die Haut eines Lebewesens eingebracht wird.
